# EUROPÄISCHE PATENTSCHRIFT

(11) **EP 0 699 699 B1**
(45) Veröffentlichungstag und Bekanntmachung des Hinweises auf die Patenterteilung: **03.11.1999**
(21) Anmeldenummer: 95112830.5
(22) Anmeldetag: 16.08.1995
(51) Int. Cl.: C08G 61/10, H05B 33/14, C07C 17/12, C07C 25/22

(54) **Poly(4,5,9,10-tetrahydropyren-2,7-diyl)-Derivate und ihre Verwendung als Elektrolumineszenzmaterialien**
Poly(4,5,9,10-tetrahydropyrene-2,7-diyl)-derivatives and their use as electroluminiscent materials
Dérivés de poly(4,5,9,10-tetrahydropyrène-2,7diyl) et leur utilisation comme matériaux électroluminiscents

(30) Priorität: 01.09.1994 DE 4431039
(43) Veröffentlichungstag der Anmeldung: 06.03.1996
(73) Patentinhaber: Aventis Research & Technologies GmbH & Co. KG, 65926 Frankfurt am Main (DE)
(72) Erfinder: Stern, Roland, Dr., D-65189 Wiesbaden (DE); Lupo, Donald, Dr., D-60316 Frankfurt (DE); Salbeck, Josef, Dr., D-65779 Kelkheim (DE); Schenk, Hermann, Dr., D-65719 Hofheim (DE); Stehlin, Thomas, Dr., D-65830 Kriftel (DE); Müllen, Klaus, Prof., D-55128 Mainz (DE); Scherf, Ullrich, Dr., D-55252 Mainz-Kastel (DE); Huber, Joachim, Dr., D-55218 Ingelheim (DE)

(56) Entgegenhaltungen:
- EP-A- 0 443 861
- CHEMICAL ABSTRACTS, Band 122, 2.-16. Jänner, 1995, Columbus, Ohio, USA M. KREYENSCHMIDT et al. "A New Soluble Poly(p-pheny- lene) with Tetrahydropyrene Repeating Units" Seite 11, Nr. 315 242x; & Macromolecules 1995, 28(13), 4577-82

## Beschreibung

Es besteht ein hoher industrieller Bedarf an großflächigen Festkörper-Lichtquellen für eine Reihe von Anwendungen, überwiegend im Bereich von Anzeigeelementen, der Bildschirmtechnologie und der Beleuchtungstechnik. Die an diese Lichtquellen gestellten Anforderungen können zur Zeit von keiner der bestehenden Technologien völlig befriedigend gelöst werden.

Als Alternative zu herkömmlichen Anzeige- und Beleuchtungselementen, wie Glühlampen, Gasentladungslampen und nicht selbstleuchtenden Flüssigkristallanzeigeelementen, sind bereits seit einiger Zeit Elektrolumineszenz(EL)materialien und -vorrichtungen, wie lichtemittierende Dioden (LED), in Gebrauch.

Neben anorganischen sind seit etwa 30 Jahren auch niedermolekulare organische Elektrolumineszenzmaterialien und -vorrichtungen bekannt (siehe z.B. US-A-3,172,862). Bis vor kurzem waren aber solche Vorrichtungen in ihrer praktischen Verwendbarkeit stark eingeschränkt.

In WO 90/13148 und EP-A 0 443 861 sind Elektrolumineszenzvorrichtungen beschrieben, die einen Film aus einem konjugierten Polymer als lichtemittierende Schicht (Halbleiterschicht) enthalten. Solche Vorrichtungen bieten zahlreiche Vorteile, wie die Möglichkeit, großflächige, flexible Displays einfach und kostengünstig herzustellen. Im Gegensatz zu Flüssigkristalldisplays sind Elektrolumineszenzdisplays selbstleuchtend und benötigen daher keine zusätzliche rückwärtige Beleuchtungsquelle.

Eine typische Vorrichtung nach WO 90/13148 besteht aus einer lichtemittierenden Schicht in Form eines dünnen, dichten Polymerfilms (Halbleiterschicht), der wenigstens ein konjugiertes Polymer enthält. Eine erste Kontaktschicht steht in Kontakt mit einer ersten Oberfläche, eine zweite Kontaktschicht mit einer weiteren Oberfläche der Halbleiterschicht. Der Polymerfilm der Halbleiterschicht hat eine genügend geringe Konzentration von extrinsischen Ladungsträgern, so daß beim Anlegen eines elektrischen Feldes zwischen den beiden Kontaktschichten Ladungsträger in die Halbleiterschicht eingebracht werden, wobei die eine Kontaktschicht positiv gegenüber der anderen wird, und die Halbleiterschicht Strahlung aussendet. Die in solchen Vorrichtungen verwendeten Polymere sind konjugiert. Unter konjugiertem Polymer versteht man ein Polymer, das ein delokalisiertes Elektronensystem entlang der Hauptkette besitzt. Das delokalisierte Elektronensystem verleiht dem Polymer Halbleitereigenschaften und gibt ihm die Möglichkeit, positive und/oder negative Ladungsträger mit hoher Mobilität zu transportieren.

In WO 90/13148 wird als polymeres Material für die lichtemittierende Schicht Poly(p-phenylenvinylen) verwendet, und es wird vorgeschlagen, die Phenylgruppe in einem solchen Material durch ein heterocyclisches oder ein kondensiertes carbocyclisches Ringsystem zu ersetzen. Daneben wird auch Poly(p-phenylen), PPP, als elektrolumineszierendes Material verwendet (G. Grem, G. Leditzky, B. Ullrich, G. Leising, Synth. Met. 1992, 51, Seite 383). Ein Hauptproblem bei der Synthese und Verarbeitung von PPP ist darin zu sehen, daß dieser Stoff schon bei sehr geringen Polymerisationsgraden Unlöslichkeit und Unschmelzbarkeit zeigt. Über sogenannte Precursor-Routen (siehe z.B. D. G. H. Ballard et al, J. Chem. Soc. Chem. Comm. 1983, Seite 954) gelang es, höher molekulares PPP zu synthetisieren und auf der Stufe eines Präpolymers zu verarbeiten. Jedoch zeigen diese Materialien unvollständige Aromatisierung und/oder ortho-Verknüpfungen sowie andere Strukturdefekte. Um die Verarbeitbarkeit des PPP zu erhöhen und die Synthese von Material mit höherem Polymerisationsgrad zu ermöglichen, wurden bereits Derivate mit Alkyl- bzw. Alkoxyseitenketten dargestellt (F. L. Klavetter, G. G. Gustafsson, A. J. Heeger, PMSE-Meeting Chicago 1993, Vol. 69, 153), die eine erhöhte Löslichkeit aufweisen. Hierbei führen die Alkyl- bzw. Alkoxyseitenketten zu einer starken Verdrillung der Phenyleinheiten und damit zu einer drastischen Abnahme der π-Orbitalüberlappung längs der Polymerkette. Ansätze, diesen Effekt zu vermeiden, basieren auf der Polymerisation von 9,10-Dihydrophenanthrenen und Fluorenen. Bei der Polymerisation von 9-Alkyl- bzw. 9,9-Dialkylfluorenen durch oxidative Kupplung mit FeCl₃ konnten Materialien mit vergleichsweise hohem Molekulargewicht (Polymerisationsgrade in der Größenordnung von 10 Repetitionseinheiten) erhalten und als aktive Schicht in Elektrolumineszenz-Vorrichtungen eingesetzt werden (M. Fukada, K. Sawada, K. Yoshino, J. Polym. Sci. Part A, Polymer Chemistry 1993, 31, Seite 2465; Y. Ohmori, M. Uchida, K. Muro, K. Yoshino, Jpn. J. Appl. Phys. 1991, 30, Seite L1941). Es wurde jedoch beobachtet, daß hierbei Fehlstellen durch Verknüpfung in den Positionen 3 und 6 auftreten.

Obwohl mit diesen Materialien gute Ergebnisse erzielt wurden, ist beispielsweise die Farbreinheit noch unbefriedigend. Weiterhin ist es mit den bisher bekannten Polymeren kaum möglich, eine blaue oder weiße Emission zu erzeugen.

Da zudem die Entwicklung von Elektrolumineszenzmaterialien, insbesondere auf Grundlage von Polymeren, noch in keiner Weise als abgeschlossen betrachtet werden kann, sind die Hersteller von Beleuchtungs- und Anzeigevorrichtungen an den unterschiedlichsten Elektrolumineszenzmaterialien für solche Vorrichtungen interessiert.

Dies liegt unter anderem auch daran, weil erst das Zusammenwirken der Elektrolumineszenzmaterialien mit den weiteren Bauteilen der Vorrichtungen Rückschlüsse auf die Qualität auch des Elektrolumineszenzmaterials zuläßt.

Aufgabe der vorliegenden Erfindung war es daher, neue Elektrolumineszenzmaterialien bereitzustellen, die bei Verwendung in Beleuchtungs- oder Anzeigevorrichtung geeignet sind, das Eigenschaftsprofil dieser Vorrichtungen zu verbessern.

Es wurde nun überraschend gefunden, daß bestimmte Derivate des Poly(4,5,9,10-tetrahydropyren-2,7-diyl) neben einer verbesserten Löslichkeit in organischen Solventien und verbesserten Filmbildungseigenschaften insbesondere auch gute Elektro- und Photolumineszenz mit einer hohen Farbreinheit aufweisen.

Gegenstand der Erfindung sind daher Verbindungen der Formel (I), 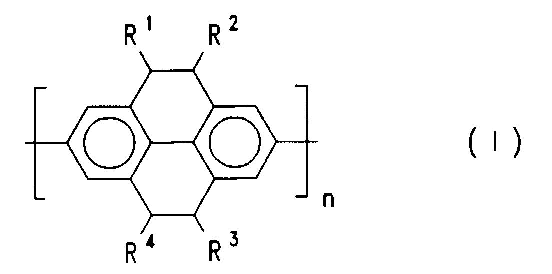 wobei die Symbole R¹ bis R⁴ folgende Bedeutungen haben:
- R¹, R², R³, R⁴: sind gleich oder verschieden H, eine geradkettige oder verzweigte Alkylkette mit 1 bis 22 C-Atomen, wobei eine oder mehrere nicht benachbarte CH₂-Gruppe auch durch -O-, -COO-, -OOC- und/oder Phenylen ersetzt sein können, Aryl- oder Aryloxygruppen, vorzugsweise mit 4 bis 10 Kohlenstoffatomen, wobei der Aromat mit C₁-C₂₂-Alkyl, C₁-C₂₂-Alkoxy, Br, Cl, F, CN, und/oder NO₂ substituiert sein kann, Br, Cl, F, CN, NO₂, oder Carboalkoxy mit 2 bis 23 C-Atomen;
- n: ist 10 bis 150.

Bevorzugt sind erfindungsgemäße Verbindungen der Formel (I), bei denen R² und R⁴ Wasserstoff bedeuten.

Weiterhin bevorzugt sind Verbindungen der Formel (I), bei denen R¹ gleich R³ ist.

Besonders bevorzugt sind erfindungsgemäße Verbindungen, bei denen R² und R⁴ Wasserstoff bedeuten sowie R¹ und R³ gleich sind.

Insbesondere bevorzugt sind erfindungsgemäße Verbindungen der Formel (I), bei denen R² und R⁴ Wasserstoff bedeuten und bei denen R¹ und R³ gleich sind und eine geradekettige oder verzweigte Alkyl- oder Alkoxygruppe mit 4 bis 12 C-Atomen, eine Alkylarylgruppe oder eine Carboalkoxygruppe mit 5 bis 13 C-Atomen bedeuten.

Die erfindungsgemäßen Verbindungen der Formel (I) sind Homo- oder Copolymere, d.h., daß das Polymer der Formel (I) auch unterschiedliche Wiederholeinheiten aufweisen kann.

Die erfindungsgemäßen Verbindungen der Formel (I) sind zur Erzielung blauer, gelber und weißer Elektrolumineszenz gut geeignet.

Vorteile der erfindungsgemäßen Polymere sind unter anderem die niedrige Kristallisationstendenz und die guten Filmbildungseigenschaften.
Die erfindungsgemäßen Polymere zeichnen sich weiterhin durch eine beträchtliche Steigerung der Löslichkeit in organischen Solventien aus. Sie sind zudem strukturell einheitlich, trotz der löslichkeitsfördernden Seitengruppen sterisch nicht zusätzlich gehindert und weisen ein vergleichsweise hohes Molgewicht auf.

Die Herstellung der erfindungsgemäßen Polymer kann nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben werden, erfolgen.
Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch gemacht werden.

Als Ausgangsverbindungen für die Herstellung der erfindungsgemäßen Polymere kommen im allgemeinen zwei Klassen von Monomeren in Frage, zum einen 4,5,9,10-Tetrahydropyrene, die gegebenenfalls in den Positionen 4,5,9,10 substituiert sind, zum anderen 2,7-difunktionalisierte 4,5,9,10-Tetrahydropyrene, die gegebenenfalls in den Positionen 4,5,9,10 substituiert sind.

Literaturbekannte Methoden zur Synthese der ersten Klasse von Monomeren, beruhen z.B. auf Übergangsmetall katalysierten Hydrierungen von Pyren [a) M. Minabe, K. Nakada, Bull. Chem. Soc. Jpn. 1985, 58, S. 1962; b) P. P. Fu, H. M. Lee, R. G. Harvey, J. Org. Chem. 1980, 45, S. 2797; c) R. G. Harvey, P. W. Rabideau, Tetrahedron Lett. 1979, S. 3695]. Weitere Synthesewege für Tetrahydropyren sind die Cyclophanroute [a) T. Sato, M. Wakabayashi, Y. Okamura, Bull. Chem. Soc. Jpn. 1967, 40, S. 2365; b) T. Yamato, S. Ide, K. Tokuhisa, M. Toshiro, J. Org. Chem. 1992, 57, S. 271] und die photochemische Cyclisierung von Distyrylbiphenyl [a) A. Padwa, A. Mazzu, Tetrahedron Lett. 1974, S. 4471; b) P. H. G. op het Veld, J. C. Langendamm, W. H. Laarhoven, Tetrahedron Lett. 1975, S. 231].
Die Literatur bietet auch Beispiele für die Einführung von Substituenten in den Positionen 4,9, wie die Photocyclisierung substituierter Distyrylbiphenyle. Nach dieser Methode werden 4,5,9,10-Tetrahydropyrene hergestellt, die symmetrisch in den Positionen 4 und 9 substituiert sind. Bei den Substituenten handelt es sich um substituierte und unsubstituierte Aryle [a) P. H. G. op het Veld, J. C. Langendamm, W. H. Laarhoven, Tetrahedron Lett. 1975, S. 231; b) W. H. Laarhoven, Th. J. H. M. Cuppen, J. C. S. Perkin 1 1972, S. 2074; c) P. H. G. op het Veld, W. H. Laarhoven, J. C. S. Perkin 2 1977, S. 268; d) P. H. G. op het Veld, W. H. Laarhoven, J. C. S. Perkin 2 1977, S. 922; e) J. Meinwald, J. W. Yound, J. Am. Chem. Soc. 1971, 93, S. 725], Carbonsäureester und Cyanogruppen [a) A. Padwa, C. Doubleday, A. Mazzu, J. Org. Chem. 1977, Vol. 42, S. 3271; b) A. Padwa, A. Mazzu, Tetrahedron Lett. 1974, S. 4471]. Ausgehend von Pyren werden weiterhin 4,5,9,10-Tetrahydroxy-4,5,9,10-tetrahydropyren [a) R. M. Moriarty, P. Dansette, D. M. Jerina, Tetrahedron Lett. 1975, 30, S. 2557] und 4,5,9,10-Pyreneterone [a) E. Clar, H. Becker, M. Correl, H. Streeck, Ann. 1937, 531, S. 1; b) J. K. Stille, E. L. Mainen, Macromolecules 1968, 1, S. 36] sowie 4,5,9,10-Pyrenbisoxiran synthetisiert.

Durch die oben erwähnte Photocyclisierung werden aus den entsprechenden Dialkylvinylbiphenylderivaten 4,5,9,10-Tetrahydropyrene mit Substituenten in den Positionen 4,9 synthetisiert. Die Methode bietet die Voraussetzung, eine Vielfalt von Substituenten in den Positionen 4 und 9 einzuführen, wie die synthetisierten Beispielen zeigen. Dabei können die entsprechenden Dialkylvinylbiphenylderivate sowohl aus Diphenaldehyd und einem Phosphoniumsalz als auch aus 2,2'-Bis(triphenylphosphoniomethyl)-biphenyldibromid und einem geeignetem Aldehyd durch die bekannten Wittig Reaktionen hergestellt werden. Um Substituenten auch in den Positionen 5 und 10 einzuführen, wird nicht von Diphenaldehyd, sondern vom entsprechenden Diketon, das ebenfalls variable Substituenten enthalten kann, ausgegangen und dieses mit Methyltriphenylphosphoniumbromid zum Dialkylvinylbiphenylderivat umgesetzt, das dann zum 5,10-disubstituierten Tetrahydropyren photocyclisiert wird. Es ist weiterhin möglich, durch die Umsetzung des angeführten Diketons mit verschiedenen Phosphoniumsalzen ein Produkt herzustellen, das zu einem vierfach substituierten Tetrahydropyren umgesetzt werden kann. Dabei kann auf diese Art und Weise durch die verschiedene Substituenten eine Asymmetrie in das Molekül gebracht werden.
Ein weiterer Weg, zwei- oder vierfach substituierte 4,5,9,10-Tetrahydropyrenderivate aufzubauen, verläuft über Diole oder Tetraole, indem man diese zu den jeweiligen Ethern, Estern oder anderen Funktionalisierungen umsetzt.

Es ist ebenfalls möglich, Substituenten einzuführen, indem man das entsprechende Tetrahydropyren in den Benzylpositionen bromiert und z.B. Alkylgruppen durch Umsetzung mit Alkylgrignardverbindungen unter Nickelkatalyse oder durch die Umsetzung mit den entsprechenden Boronsäuren nach Suzuki oder Miller (siehe unten) einführt.

Zur Herstellung der zweiten Klase von Monomeren müssen Funktionen in die 2 und 7 Position des Tetrahydropyrens eingeführt werden.
Dazu sind zwei Synthesewege bevorzugt, die beispielhaft in den Schemata 1 und 2 dargestellt sind.

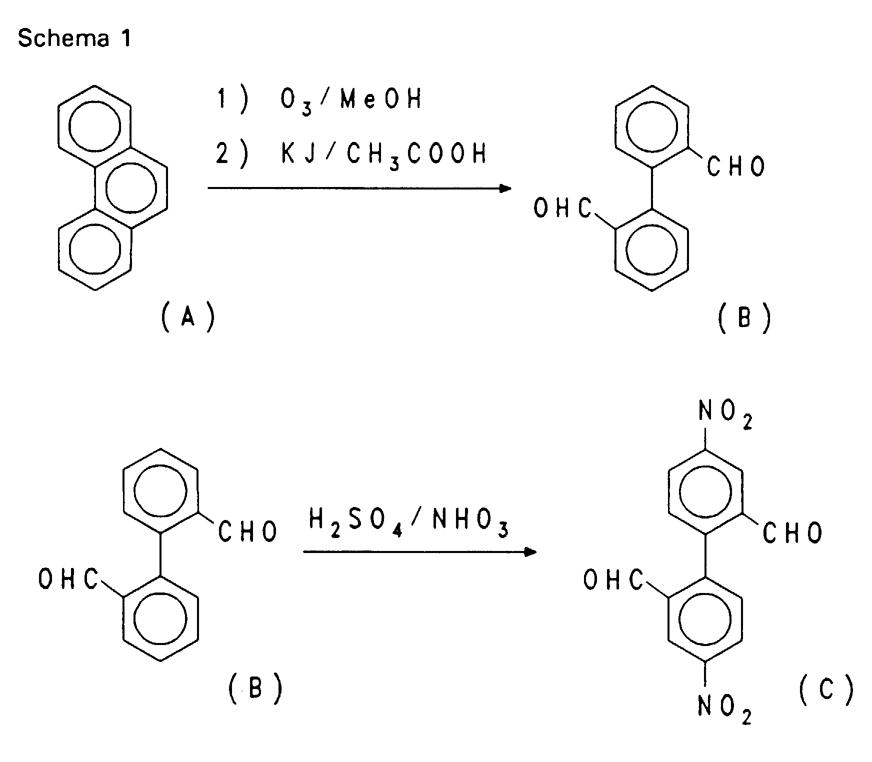

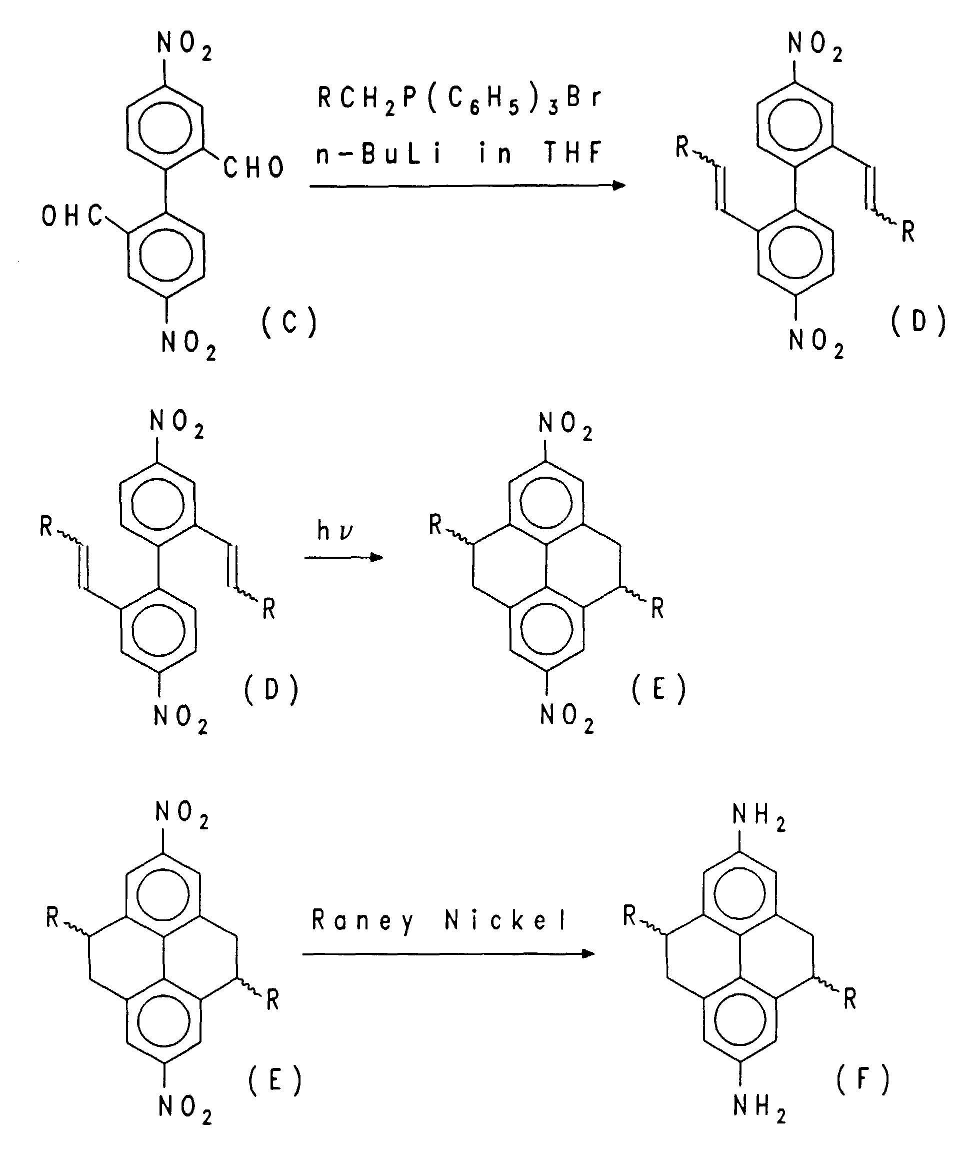

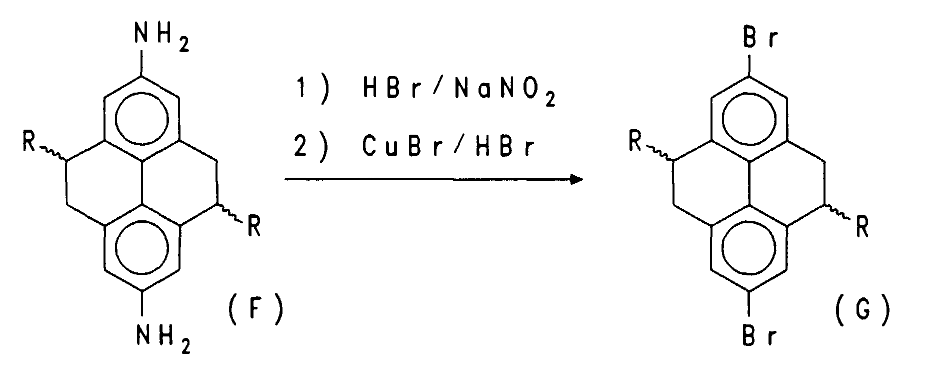

Nach Schema 1 wird Phenanthren (A) durch Ozonolyse und anschließende Oxidation des Ozonids mit Natriumiodid zum Diphenaldehyd (B) umgesetzt. Dieser läßt sich mit HNO₃/H₂SO zur Dinitroverbindung (C) nitrieren, die dann durch eine Wittig Reaktion in das 4,4'-Dinitro-2,2'-divinylbiphenyl Derivat (D) überführt wird. (D) wird photochemisch zum Tetrahydropyren Derivat (E) cyclisiert.
Dieses Zwischenprodukt erlaubt eine variable Funktionalisierung in den Positionen 2 und 7, da die Nitrogruppe durch eine Vielzahl anderer funktioneller Gruppen ersetzt werden kann. Als Beispiel ist in Schema 1 die Synthese der Dibromverbindung (G) durch Reduktion der Dinitroverbindung (E) zum Diamin (F) und anschließende Sandmeyer Reaktion gezeigt.

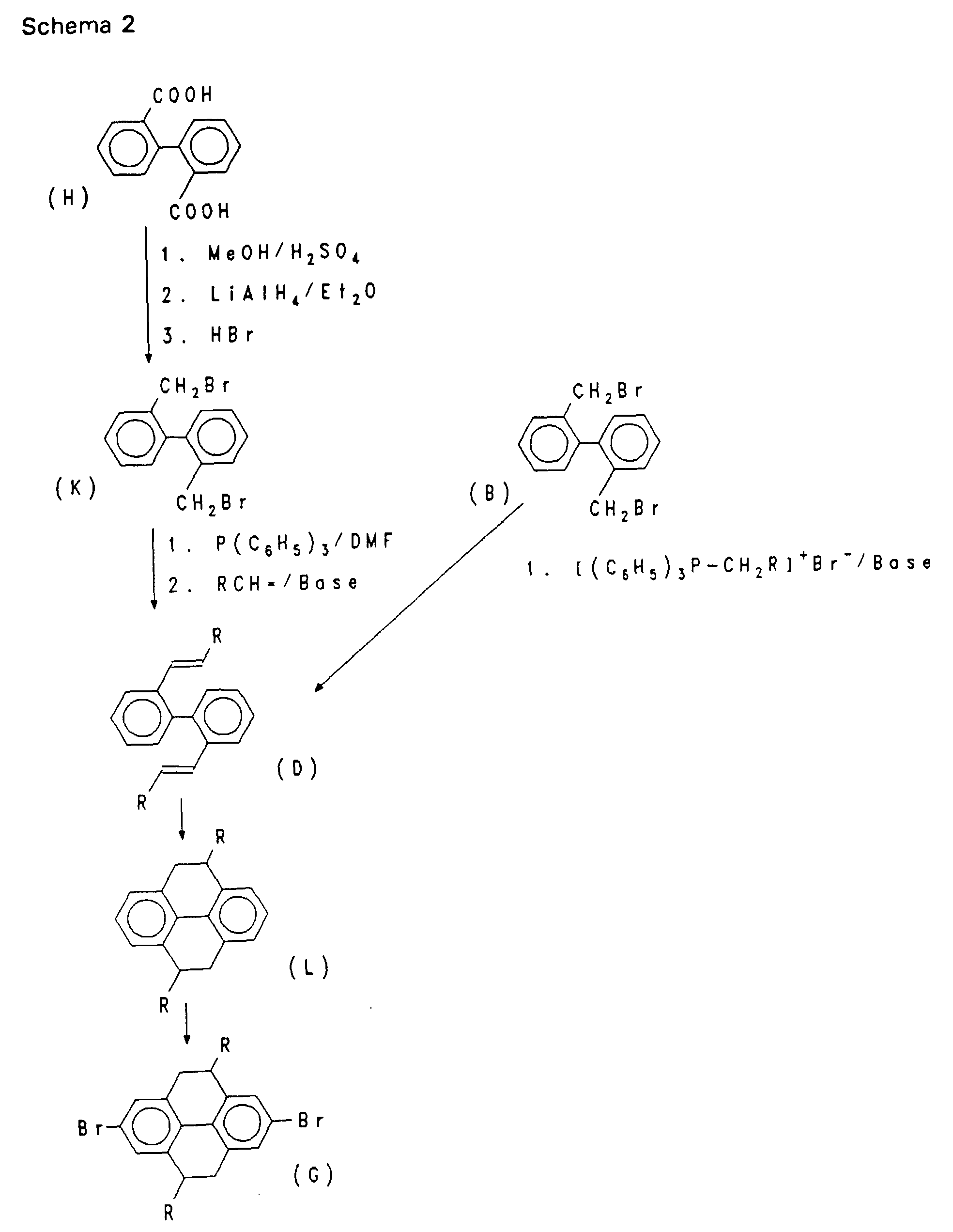

Ein zweiter Weg zu 2,7-difunktionalisierten 4,5,9,10-Tetrahydropyren-Derivaten ist bespielhaft in Schema 2 dargestelt.

Diphensäure (H) wird mit Methanol zum Dimethylester umgesetzt, der mit Lithiumaluminiumhydrid zum 2,2'-Bis-hydroxymethylbiphenyl reduziert wird. Substitution der Hydroxygruppen durch Brom mittels HBr führt dann zu Dibromverbindung (K). Aus (K) wird durch Umsetzung mit Triphenylphosphin ein Bisphosphoniumsalz hergestellt, das nach Deprotonierung zum Phosphoran mit Aldehyden zur Divinylverbindung (D) reagiert. Alternativ kann (B) auch aus dem in Schema 1 beschriebenen Diphenaldehyd (B) durch Wittig Reaktion hergestellt werden. Die Vinylverbindung (D) wird durch Photocyclisierung zum 4,5,9,10-Tetrahydropyrenderivat (L) umgesetzt.
Daraus kann nach einer neuen Bromierungsmethode, die ebenfalls Gegenstand der Erfindung ist, die oben beschriebene Dibromverbindung (G) hergestellt werden. Das neue Verfahren zur Herstellung von 2,7-Dibrom-4,5,9,10-tetrahydropyren-Derivaten ist dadurch gekennzeichnet, daß ein 4,5,9,10-Tetrahydropyrenderivat in einem inerten organischen Lösungsmittel in Gegenwart eines Metalls der achten Nebengruppe auf Aktivkohle als Trägermaterial mit Brom umgesetzt wird.

Das erfindungsgemäße Verfahren zeichnet sich insbesondere durch seine ausgezeichnete Selektivität aus; es wird ausschließlich in den Positionen 2 und 7 bromiert.

Das Verfahren wird im allgemeinen bei einer Temperatur von -50 bis 100°C, vorzugsweise 0 bis 50°C, besonders bevorzugt bei Raumtemperatur, durchgeführt.

Bevorzugt sind stark polare, protische oder aprotische Lösungsmittel, wie DMF und Wasser.

Als Katalysator dient bei dem erfindungsgemäßen Verfahren ein Metall der achten Nebengruppe auf Aktivkohle.

Als Metall der achten Nebengruppe wird vorzugsweie Palladium, Platin, Iridium oder Nickel, besonders bevorzugt Palladium oder Platin, insbesondere Palladium verwendet.
Bevorzugte Katalysatoren enthalten etwa 5 bis 10 Gew.-% Metall auf der Aktivkohle.
Der Katalysator wird vorzugsweise in einer Menge von 0,2 bis 20 Mol-%, besonders bevorzugt von 1 bis 4 Mol-%, bezogen auf das Tetrahydropyrenderivat, eingesetzt.
Vorzugsweise werden 0,9 bis 3 Äquivalente Brom pro Bromfunktion im Zielmolekül verwendet.

Die Aufarbeitung erfolgt nach bekannten, dem Fachmann geläufigen Methoden. Beispielsweise zerstört man überschüssiges Brom durch Zugabe von wäßriger NaOH- oder NaHSO₃-Lösung, verdünnt mit Wasser, extrahiert mit einem wasserunlöslichen organischen Lösungsmittel und reinigt das nach Trocknen und Abziehen des Lösungsmittels gewonnene Rohprodukt durch Chromatographie.

Die nach diesem Verfahren hergestellten 2,7-Dibrom-4,5,9,10-tetrahydropyren-Derivate eignen sich hervorragend als Ausgangsprodukte zur Herstellung erfindungsgemäßer Polymere der Formel (I).

Sie können aber ebensogut nach literaturbekannten Methoden beispielsweise zu Diboronsäuren (M. Miyaura, T. Yanagi, A. Suzuki, Synth. Commun. 1981, 11, 513; R. B. Miller, S. Dugar, Organometallics 1984, 3, 1261), gemischten Brom-Boronsäuren (M. Rehahn, A. D. Schlüter, G. Wegner, W. J. Feast, Polymer 1989, 30, 1054) oder Distannanen (J. K. Stille, Angew. Chem. Int. Ed. Engl. 1986, 25, 508) umgesetzt werden, die ebenfalls Ausgangsverbindungen für die erfindungsgemäßen Polymere sind.

Die Herstellung der erfindungsgemäßen Polymere der Formel (I) ist nach mehreren Methoden möglich.
Beispielsweise können Derivate des 4,5,9,10-Tetrahydropyrens oxidativ (z.B. mit FeCl₃, siehe u.a. P. Kovacic, N. B. Jones, Chem. Ber. 1987, 87, 357 bis 379; M. Weda, T. Abe, H. Awano, Macromolecules 1992, 25, 5125) oder elektrochemisch (siehe z.B. N. Saito, T. Kanbara, T. Sato, T. Yamamoto, Polym. Bull. 1993, 30, 285) polymerisiert werden.

Ebenso können die erfindungsgemäßen Polymere der Formel (I) aus 2,7-difunktionalisierten 4,5,9,10-Tetrahydropyren-Derivaten hergestellt werden. Dihalogenaromaten lassen sich unter Kupfer/Tripenylphosphin - (siehe z.B. G. W. Ebert, R. D. Rieke, J. Org. Chem. 1988, 53, 44829) oder Nickel/Triphenylphosphin-Katalyse (siehe z.B. H. Matsumoto, S. Inaba, R. D. Rieke, J. Org. Chem. 1983, 48, 840) polymerisieren.

Aromatische Diboronsäuren und aromatische Dihalogenide oder gemischte aromatische Halogen-Boronsäuren lassen sich unter Palladiumkatalyse durch Kupplungsreaktionen polymerisieren (siehe z.B. M. Miyaura, T. Yanagi, A. Suzuki, Synth. Commun. 1981, 11, 513; R. B. Miller, S. Dugar, Organometallics 1984, 3, 1261).

Aromatische Distannane lassen sich z.B., wie bei J. K. Stille, Angew. Chem. Int. Ed. Engl. 1986, 25, 508 angegeben, unter Palladiumkatalyse polymerisieren.

Weiterhin können die oben erwähnten Dibromverbindungen in die Dilithio- oder Digrignardverbindungen übergeführt werden, die dann mit weiterer Dibromverbindung mittels CuCl₂ (siehe z.B. G. Wittig, G. Klar, Liebigs Ann. Chem. 1967, 704, 91; H. A. Stabb, F. Bunny, Chem. Ber. 1967, 100, 293; T. Kaufmann, Angew. Chem. 1974, 86, 321 bis 354) oder durch Elektronentransfer ungesättigter 1,4-Dihalogenverbindungen (siehe z.B. S. K. Taylor, S. G. Bennett, K. J. Harz, L. K. Lashley, J. Org. Chem. 1981, 46, 2190) polymerisiert werden.

Bevorzugt ist ein Verfahren zur Herstellung von Polymeren der Formel (I), 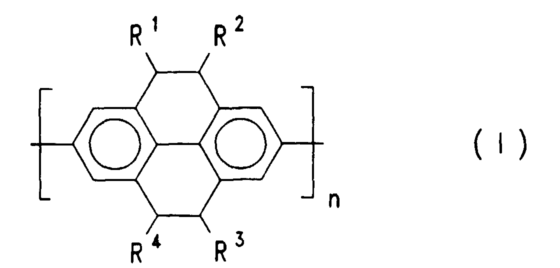 wobei die Symbole R¹ bis R⁴ folgende Bedeutungen haben:
- R¹, R², R³, R⁴: sind gleich oder verschieden H, eine geradkettige oder verzweigte Alkylkette mit 1 bis 22 C-Atomen, wobei eine oder mehrere nicht benachbarte CH₂-Gruppe auch durch -O-, -COO-, -OOC- und/oder Phenylen ersetzt sein können, Aryl- oder Aryloxygruppen, wobei der Aromat mit C₁-C₂₂-Alkyl, C₁-C₂₂-Alkoxy, Br, Cl, F, CN, und/oder NO₂ substituiert sein kann, Br, Cl, F, CN, NO₂, oder Carboalkoxy mit 2 bis 23 C-Atomen und
- n: ist 10 bis 150,
das dadurch gekennzeichnet ist, daß man eine oder mehrere Verbindungen der Formel (II), 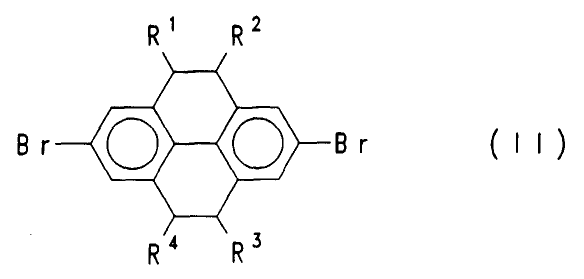 worin die Symbole dieselben Bedeutungen wie in der Formel (I) haben, in einem inerten organischen Lösungsmittel oder Lösungsmittelgemisch mit Bis(1,5-cyclooctadien)nickel(0) und einem 2,2'-Bipyridin umsetzt.

Die Umsetzung erfolgt im allgemeinen bei einer Temperatur von 0 bis 150°C, vorzugsweise 20 bis 100°C, besonder bevorzugt 40 bis 90°C. Die Reaktionsdauer liegt im allgemeinen bei 1 bis 7, üblicherweise 2 bis 3 Tagen.

Bevorzugte Lösungsmittel sind N,N-Dialkylamide, wie Dimethylformamid (DMF), Ether, wie Tetrahydrofuran (THF), aromatische Kohlenwasserstoffe, wie Toluol, und Gemische der angeführten Lösungsmittel.
Besonders bevorzugt ist eine Mischung aus DMF und Toluol, insbesondere im Verhältnis 1:3.
Bis(1,5-Cyclooctadien)nickel(0) (Ni(COD)₂) kann direkt eingesetzt werden, es kann aber auch in situ in der Reaktionsmischung aus 1,5-Cyclooctadien und einem Nickelkomplex, wie Ni(dppp)Cl₂, Ni(acac)₂, Ni(bipy)Cl₂ oder Ni(PPh₃)₂Cl₂ gebildet werden. Vorzugsweise enthält die Reaktionsmischung in jedem Fall einen Überschuß von Cyclooctadien.
Das eingesetzte 2,2'-Bipyridin kann gegebenenfalls substituiert sein; es wird im allgemeinen im Überschuß, bezogen auf den Nickelkomplex, eingesetzt.
Im allgemeinen werden 0,9 bis 2, vorzugsweise 1 bis 1,5, besonders bevorzugt 1,2 bis 1,3 Äquivalente des Kupplungsreagenzes (NiCoD₂ (2,2-Dipyridin) pro Bromatom verwendet.

Zur Herstellung von Copolymeren können beispielsweise unterschiedliche Verbindungen der Formel (II) gemeinsam polymerisiert werden.

Die Aufarbeitung erfolgt nach bekannten, dem Fachmann geläufigen Methoden. Beispielsweise kann man die Reaktionsmischung filtrieren, mit wäßriger Säure verdünnen, extrahieren und das nach Trocknen und Abziehen des Lösungsmittels erhaltene Rohprodukt durch Umfällen weiter reinigen.

Endständige Bromatome können beispielsweise mit LiAlH₄ reduktiv entfernt werden.

Die erfindungsgemäßen Polymere können als Elektrolumineszenzmaterialien Verwendung finden, d.h., sie dienen als aktive Schicht in einer Elektrolumineszenzvorrichtung. Als aktive Schicht im Sinne der Erfindung gelten Elektrolumineszenzmaterialien, die befähigt sind, bei Anlegen eines elektrischen Feldes Licht abzustrahlen (lichtemittierende Schicht), sowie Materialien, welche die Injektion und/oder den Transport der positiven und/oder negativen Ladungen verbessern (Ladungsinjektionsschichten und Ladungstransportschichten).

Gegenstand der Erfindung sind daher auch die Verwendung von erfindungsgemäßen Polymeren der Formel (I) als Elektrolumineszenzmaterialien sowie Elektrolumineszenzmaterialien, enthaltend ein Polymer der Formel (I).

Gegenstand der Erfindung ist weiterhin eine Elektrolumineszenzvorrichtung mit einer oder mehreren aktiven Schichten, wobei mindestens eine dieser aktiven Schichten ein oder mehrere erfindungsgemäße Polymere enthält. Die aktive Schicht kann beispielsweise eine lichtemittierende Schicht und/oder eine Transportschicht und/oder eine Ladungsinjektionsschicht sein.

Der allgemeine Aufbau solcher Elektrolumineszenzvorrichtungen ist beispielsweise in US 4,539,507 und US 5,151,629 beschrieben. Polymere enthaltende Elektrolumineszenzvorrichtungen sind beispielsweise in WO 90/13148 oder EP-A 0 443861 beschrieben.

Sie enthalten üblicherweise eine elektrolumineszierende Schicht zwischen einer Kathode und einer Anode, wobei mindestens eine der Elektroden transparent ist. Zusätzlich kann zwischen der elektrolumineszierenden Schicht und der Kathode eine Elektroneninjektions- und/oder Elektronentransportschicht eingebracht sein und/oder zwischen der elektrolumineszierenden Schicht und der Anode eine Lochinjektions- und/oder Lochtransportschicht eingebracht sein. Als Kathode können z.B. Ca, Mg, Al, In, Mg/Ag dienen. Als Anode können z.B. Au oder ITO (Indiumoxid/Zinnoxid) auf einem transparentem Substrat, z.B. aus Glas oder einem transparenten Polymer, dienen.

Im Betrieb wird die Kathode auf negatives Potential gegenüber der Anode gesetzt. Dabei werden Elektronen von der Kathode in die Elektroneninjektionsschicht/Elektronentransportschicht bzw. direkt in die lichtemittierende Schicht injiziert. Gleichzeitig werden Löcher von der Anode in die Lochinjektionsschicht/Lochtransportschicht bzw. direkt in die lichtemittierende Schicht injiziert.

Die injizierten Ladungsträger bewegen sich unter dem Einfluß der angelegten Spannung durch die aktiven Schichten aufeinander zu. Dies führt an der Grenzfläche zwischen Ladungstransportschicht und lichtemittierender Schicht bzw. innerhalb der lichtemittierenden Schicht zu Elektronen/Loch-Paaren, die unter Aussendung von Licht rekombinieren.
Die Farbe des emittierten Lichtes kann durch die als lichtemittierende Schicht verwendete Verbindung variiert werden.

Elektrolumineszenzvorrichtungen finden Anwendung z.B. als selbstleuchtende Anzeigeelemente, wie Kontrollampen, alphanumerische Displays, Hinweisschilder, und in optoelektronischen Kopplern.

Die Erfindung wird durch die Beispiele näher erläutert.

### Beispiele

### Beispiel 1

Cis-trans-lsomere des 2,2'-Dodecyl-1"-en-biphenyl 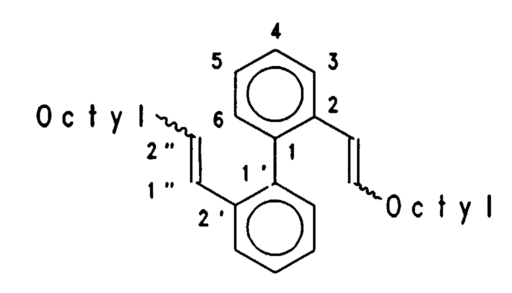
Summenformel: C₃₂H₄₆
Molgewicht: 430,5 g/mol
Schmelzpunkt: farbloses Öl

### Synthesebeschreibung

In einem inertisierten 1 l Dreihalskolben mit Rückflußkühler und Tropftrichter werden 84 g (0,1 mol) 2,2'-Bis(triphenylphosphoniomethyl)biphenyldibromid sowie 42,6 g (0,3 mol) Nonanal in 400 ml abs. EtOH gelöst und auf 75°C erwärmt. Innerhalb von 4 Stunden werden 500 ml 0,5 molare Natriumalkoholatlösung zugetropft, wobei durch die Bildung des Ylids eine Gelbfärbung eintritt. Es wird 20 Stunden bei 75°C nachgerührt und nach Abkühlen der Lösung und Zugabe von 800 ml H₂O dreimal mit Methylenchlorid ausgeschüttelt. Die vereinigten organischen Phasen werden über MgSO₄ getrocknet und das Lösungsmittel im Vakuum abgezogen. Die Reinigung erfolgt durch Säulenchromatographie auf Kieselgel mit Petrolether/Ether (20:1). Ausbeute: 36 g (87 %)

### Beispiel 2

Cis-trans-lsomere des 4,9-Dioctyl-4,5,9,10-tetrahydropyren 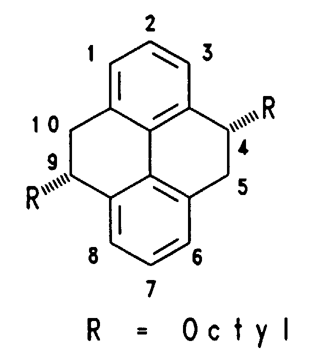
Summenformel: C₃₂H₄₆
Molgewicht: 430,5 g/mol
Schmelzpunkt: farbloses Öl

### Synthesebeschreibung

2,154 g (0,005 mol) 2,2'-Didecyl-1"-en-biphenyl werden in 300 ml n-Hexan gelöst und in einen Photoreaktor gefüllt. Nun wird unter Rühren 30 Minuten lang ein Argonstrom durch die Lösung geleitet, um den Restsauerstoff möglichst zu entfernen. Darauf wird unter einem leichten Argonstrom bei einer Wellenlänge von 254 nm (80 Watt) mit einer Hg-Niederdruck-Tauchlampe der Fa. Gräntzel, zwischen 5 und 6 Stunden bestrahlt. Das Ende der Photoreaktion wird durch ¹H-NMR ermittelt. Das Lösungsmittel wird am Rotationsverdampfer abgezogen, wobei ein gelbliches Öl zurückbleibt, das auf Kieselgel mit Petrolether/Ether (20:1) chromatographiert wird. Ausbeute: 2,01 g (94 %)

### Beispiel 3

Cis- oder trans-2,7-Dibrom-4,9-dioctyl-4,5,9,10-tetrahydropyren 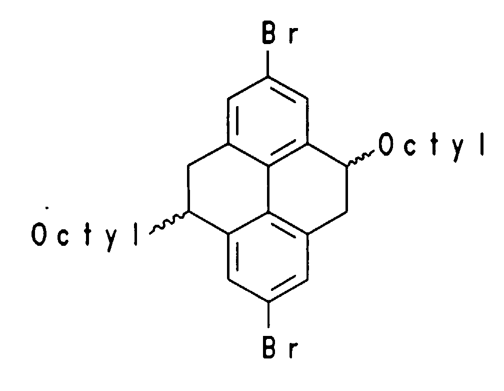
Summenformel: C₃₂H₄₄Br₂
Molgewicht: 588,5 g/mol
Schmelzpunkt: Öl

### Synthesebeschreibung

In einem 250 ml Zweihalskolben mit Tropftrichter werden 10,75 g (0,025 mol) 4,9-Dioctyl-4,5,9,10-tetrahydropyren in 170 ml DMF gelöst, 100 mg 5 %iges Palladium auf Aktivkohle zugegeben und unter Rühren bei Raumtemperatur und Lichtausschluß 11,98 g (3,85 ml, 0,075 mol) Brom in 40 ml DMF innerhalb von 2 Stunden zugetropft. Man läßt 12 bis 16 Stunden nachreagieren; daraufhin zerstört man überschüssiges Brom durch Zugabe einer 15 gew.-%igen NaOH-Lösung. Es werden 200 ml Wasser zugegeben und dreimal mit 50 ml Methylenchlorid exthrahiert. Zum Abtrennen des Palladium-Katalysators wird über Celite® (Filterhilfsmittel der Fa. Aldrich, Steinheim) filtriert, anschließend über Magnesiumsulfat getrocknet und das Lösungsmittel abgezogen. Das gelbe Öl wird zweimal über Aluminiumoxid und zweimal über Kieselgel jeweils mit Petrolether chromatographiert. Das erste Isomere (trans) reichert sich jeweils in den ersten Fraktionen an und fällt zu 52 % (7,65 g) als farbloses Öl, das nach einigen Tagen weiß auskristallisiert, an.
Die zweite Isomerenfraktion kann zu 33 % ebenfalls als farbloses Öl gewonnen werden, das auch nach einigen Tagen auskristallisiert.

### Beispiel 4

Poly(4,9-dioctyl-4,5,9,10-tetrahydropyren-2,7-diyl) 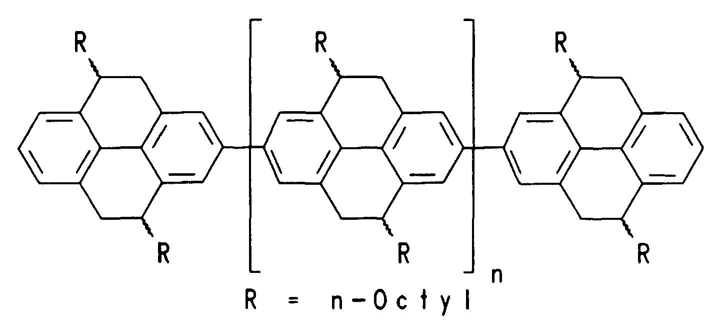
Summenformel: C₃₂ₓH₄₄ₓ
Gewicht der Monomereinheit: 428,704 g/mol
Schmelzpunkt: 270°C (TGA)

### Synthesebeschreibung

In inertisierten und mit Argon gefluteten 100 ml Schlenckrohr wird ein Kupplungsreagenz aus 1,1 g (0,00377 mol) Ni(COD)₂, 650 mg (0,00436 mol) 2,2'-Bipyridyl und 434 mg (0,35 ml) Cyclooctadien in 7 ml abs. DMF und 10 ml abs. Toluol hergestellt. Die Lösung ist tiefblau bis lila gefärbt und wird bei 70°C 30 Minuten gerührt. In einem separat entgasten 50 ml Schlenckkolben werden 1,491 g (0,00254 mol) 2,7-Dibrom-4,9-dioctyl-4,5,9,10-tetrahydropyren in 12 ml abs. Toluol gelöst, auf 50°C erwärmt und zu der Lösung des Kupplungsreagenzes zugespritzt. Die Lösung verfärbt sich nach wenigen Augenblicken rot-braun und wird 3 Tage unter Lichtausschluß bei 75°C gerührt. Man läßt abkühlen, filtriert über Celite® (Filterhilfsmittel der Fa. Aldrich, Steinheim), gibt die Lösung in 100 ml 5N wäßrige HCI und läßt 1 Stunde auf dem Magnetrührer durchmischen. Die wäßrige Lösung wird dann dreimal mit 50 ml CHCl₃ extrahiert und die vereinigten organischen Phasen dreimal mit 150 ml wäßriger EDTA-Lösung von pH = 9 und dreimal mit 150 ml wäßriger EDTA-Lösung von pH = 3,8, sowie noch zweimal mit 150 ml 5N wäßriger HCl und abschließend mehrfach mit Wasser gewaschen. Die vereinigten organischen Phasen werden über MgSO₄ getrocknet und das Lösungsmittel im Vakuum abgezogen. Zum Entfernen entständiger Bromatome wird in einem 250 ml inertisierten und mit Argon belüfteten Dreihalskolben mit Rückflußkühler und Septum eine Suspension von 5 g LiAlH₄ in 50 ml abs. THF angesetzt und das in 50 ml abs. Toluol gelöste Polymer über das Septum zugespritzt. Darauf wird 3 Tage zum Rückfluß erhitzt. Die Reaktionsmischung wird nach Erkalten im Eisbad mit 25 ml 2N H₂SO₄ hydrolysiert und darauf mit 200 ml H₂O versetzt. Die Lösung wird mehrfach mit 100 ml CHCl₃ ausgeschüttelt, die vereinigten organischen Phasen über Magnesiumsulfat getrocknet und das Lösungsmittel im Vakuum bis auf 10 ml eingeengt. Nun wird das Polymer in 100 ml Aceton ausgefällt und über eine Fritte abgesaugt. Die Farbe des Polymers ist hellgelb.

### Beispiel 5

### Elektrolumineszenz-Vorrichtung

Eine Lösung aus Poly(4,9-dioctyl-4,5,9,10-tetrahydropyren-2,7-diyl) in Chloroform (15 mg/ml) wird unter Stickstoff durch Spin-Coating bei 1000 upm auf einen mit ITO (Indium-Zinn-Oxid) beschichteten Glasträger (strukturiert, Streifen 2 mm breit) aufgebracht. Der Glasträger wird über eine Schleuse unter Beibehaltung der Schutzgasatmosphäre in eine Hochvakuum-Bedampfungsanlage überführt. Bei 2x10⁻⁵ mbar werden quer zu den ITO-Streifen unter Verwendung einer Maske Ca-Streifen (2 mm breit, 230 nm dick) auf die Polymerschicht aufgedampft. Die so erhaltene Vorrichtung, ITO/ Poly(4,9-dioctyl-4,5,9,10-tetrahydropyren-2,7-diyl)/Ca, wird in einen Probenhalter gegeben und die Elektroden über Federkontakte mit einer Stromquelle verbunden, wobei ein ITO-Streifen positiv und ein Ca-Streifen negativ gepolt werden. Beim Anlegen einer Spannung von 23 V, wird an dem entsprechenden Matrixelement eine intensive, homogene, blaue Fluoreszenz (11 cd/m² bei 0,27 mA; unter Argonatmosphäre) beobachtet. Die externe Effizienz beträgt 0,11 %. Das Elektrolumineszenzspektrum entspricht in wesentlichen dem Photolumineszenzspektrum (siehe Abb. 1; Photolumineszenz eines mittels Spin-Coating auf Quarzträger aufgebrachten Films).

## Patentansprüche

1. Poly(4,5,9,10-tetrahydropyren-2,7-diyl)-Derivate der Formel (I), 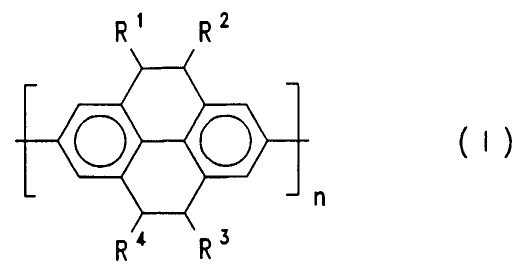 wobei die Symbole R¹ bis R⁴ folgende Bedeutungen haben:
R¹, R², R³, R⁴ sind gleich oder verschieden H, eine geradkettige oder verzweigte Alkylkette mit 1 bis 22 C-Atomen, wobei eine oder mehrere nicht benachbarte CH₂-Gruppe auch durch -O-, -COO-, -OOC- und/oder Phenylen ersetzt sein können, Aryl- oder Aryloxygruppen, wobei der Aromat mit C₁-C₂₂-Alkyl, C₁-C₂₂-Alkoxy, Br, Cl, F, CN, und/oder NO₂ substituiert sein kann, Br, Cl, F, CN, NO₂, oder Carboalkoxy mit 2 bis 23 C-Atomen;
n ist 10 bis 150.

2. Poly(4,5,9,10-tetrahydropyren-2,7-diyl)-Derivat nach Anspruch 1, dadurch gekennzeichnet, daß es ein Homopolymer ist.

3. Poly(4,5,9,10-tetrahydropyren-2,7-diyl)-Derivat nach Anspruch 1, dadurch gekennzeichnet, daß es ein Copolymer ist.

4. Poly(4,5,9,10-tetrahydropyren-2,7-diyl)-Derivate nach einem oder mehreren der Ansprüche 1 bis 3, dadurch gekennzeichnet, daß in der Formel (I) R² und R⁴ H bedeuten.

5. Poly(4,5,9,10-tetrahydropyren-2,7-diyl)-Derivate nach einem oder mehreren der Ansprüche 1 bis 4, dadurch gekennzeichnet, daß in der Formel (I)
R² und R⁴ H und
R¹ und R³ gleich eine geradkettige oder verzweigte Alkylkette mit 1 bis 22 C-Atomen, wobei eine oder mehrere nicht benachbarte CH₂-Gruppe auch durch -O-, -COO-, -OOC- und/oder Phenylen ersetzt sein können, Aryl- oder Aryloxygruppen, wobei der Aromat mit C₁-C₂₂-Alkyl, C₁-C₂₂-Alkoxy, Br, Cl, F, CN, und/oder NO₂ substituiert sein kann, Br, Cl, F, CN, NO₂, oder Carboalkoxy mit 2 bis 23 C-Atomen;
n 10 bis 150
bedeuten.

6. Verfahren zur Herstellung von Verbindungen der Formel (I) nach einem oder mehreren der Ansprüche 1 bis 5, dadurch gekennzeichnet, daß man eine oder mehrere Verbindungen der Formel (II), 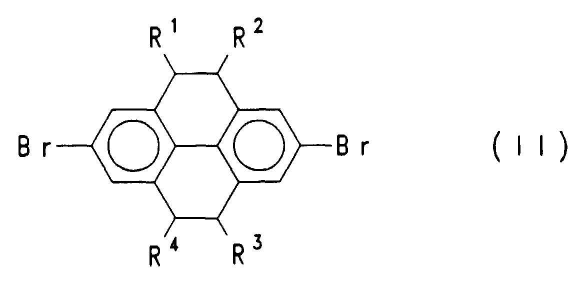 worin die Symbole dieselben Bedeutungen wie in der Formel (I) in Anspruch 1 haben, in einem inerten organischen Lösungsmittel oder Lösungsmittelgemisch mit Bis(1,5-cyclooctadien)nickel(0) und einem 2,2'-Bipyridin umsetzt.

7. Verwendung von Poly(4,5,9,10-tetrahydropyren-2,7-diyl)-Derivate nach einem oder mehreren der Ansprüche 1 bis 5 als Elektrolumineszenzmaterialien.

8. Elektrolumineszenzmaterial, enthaltend ein oder mehrere Poly(4,5,9,10-tetrahydropyren-2,7-diyl)-Derivate nach einem oder mehreren der Ansprüche 1 bis 5.

9. Elektrolumineszenzvorrichtung mit einer oder mehreren aktiven Schichten, dadurch gekennzeichnet, daß mindestens eine dieser aktiven Schichten ein Poly(4,5,9,10-tetrahydropyren-2,7-diyl)-Derivate nach einem oder mehreren der Ansprüche 1 bis 5 als Elektrolumineszenzmaterial enthält.

10. Verfahren zur Herstellung von 2,7-Dibrom-4,5,9,10-tetrahydropyren-Derivaten, dadurch gekennzeichnet, daß ein 4,5,9,10-Tetrahydropyrenderivat in einem inerten organischen Lösungsmittel in Gegenwart eines Metalls der achten Nebengruppe auf Aktivkohle als Trägermaterial mit Brom umgesetzt wird.

## Claims

1. A poly(4,5,9,10-tetrahydropyrene-2,7-diyl) derivative of the formula (I) 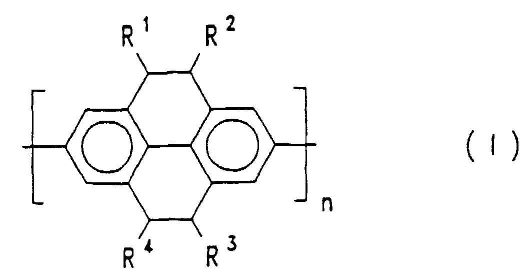 where the symbols R¹ to R⁴ have the following meanings:
R¹, R², R³, R⁴ are identically or variously H, a straight-chain or branched alkyl chain containing 1 to 22 carbon atoms, it being possible for one or more nonadjacent CH₂ groups also to be replaced by -O-, -COO-, -OOC- and/or phenylene, aryl or aryloxy groups, it being possible for the aromatic to be substituted by C₁-C₂₂-alkyl, C₁-C₂₂-alkoxy, Br, Cl, F, CN, and/or NO₂, Br, Cl, F, CN, NO₂, or carboalkoxy containing 2 to 23 carbon atoms;
n is 10 to 150.

2. A poly(4,5,9,10-tetrahydropyrene-2,7-diyl) derivative as claimed in claim 1, which is a homopolymer.

3. A poly(4,5,9,10-tetrahydropyrene-2,7-diyl) derivative as claimed in claim 1, which is a copolymer.

4. A poly(4,5,9,10-tetrahydropyrene-2,7-diyl) derivative as claimed in one or more of claims 1 to 3, wherein R² and R⁴ in the formula (I) are H.

5. A poly(4,5,9,10-tetrahydropyrene-2,7-diyl) derivative as claimed in one or more of claims 1 to 4, wherein, in the formula (I),
R² and R⁴ are H and
R¹ and R³ are identically a straight-chain or branched alkyl chain containing 1 to 22 carbon atoms, it being possible for one or more nonadjacent CH₂ groups also to be replaced by -O-, -COO-, -OOC- and/or phenylene, aryl or aryloxy groups, it being possible for the aromatic to be substituted by C₁-C₂₂-alkyl, C₁-C₂₂-alkoxy, Br, Cl, F, CN, and/or NO₂, Br, Cl, F, CN, NO₂, or carboalkoxy containing 2 to 23 carbon atoms;
n is 10 to 150.

6. A process for preparing compounds of the formula (I) as claimed in one or more of claims 1 to 5, which comprises reacting one or more compounds of the formula (II) 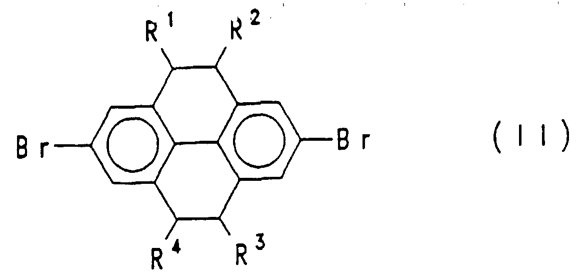 in which the symbols have the same meanings as in formula (I) in claim 1, with bis(1,5-cyclooctadiene)nickel(0) and a 2,2'-bipyridine in an inert organic solvent or solvent mixture.

7. The use of a poly(4,5,9,10-tetrahydropyrene-2,7-diyl) derivative as claimed in one or more of claims 1 to 5 as electroluminescence material.

8. An electroluminescence material containing one or more poly(4,5,9,10-tetrahydropyrene-2,7-diyl) derivatives as claimed in one or more of claims 1 to 5.

9. An electroluminescence device having one or more active layers, wherein at least one of said active layers contains a poly(4,5,9,10-tetrahydropyrene-2,7-diyl) derivative as claimed in one or more of claims 1 to 5 as electroluminescence material.

10. A process for preparing 2,7-dibromo-4,5,9,10-tetrahydropyrene derivatives, which comprises reacting a 4,5,9,10-tetrahydropyrene derivative with bromine in an inert organic solvent in the presence of a metal of group 8 on active carbon as support material.

## Revendications

1. Dérivés poly(4,5,9,10-tétrahydropyrèn-2,7-diyle) de formule (I) 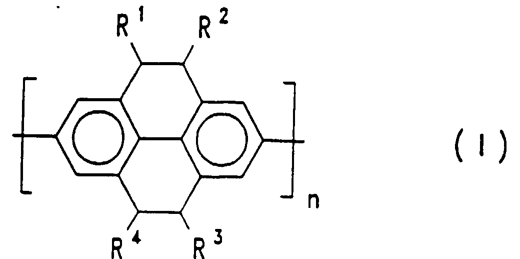 dans laquelle les symboles R¹ à R⁴ ont les significations suivantes :
R¹, R², R³, R⁴ sont identiques ou différents et représentent un atome d'hydrogène, une chaîne alkyle linéaire ou ramifiée ayant 1 à 22 atomes de carbone, dans laquelle un ou plusieurs groupes CH₂ non voisins peuvent même être remplacés par -O-, -COO-, -OOC- et/ou un groupe phénylène, des groupes aryle ou aryloxy, le composé aromatique pouvant être substitué avec un groupe alkyle en C₁-C₂₂, alcoxy en C₁-C₂₂, Br, CI, F, CN, et/ou NO₂, représentent un atome de brome, de chlore, de fluor, un groupe CN, NO₂, ou carboalcoxy avec 2 à 23 atomes de carbone;
n est 10 à 150.

2. Dérivé poly(4,5,9,10-tétrahydropyrèn-2,7-diyle) selon la revendication 1, caractérisé en ce qu'il est un homopolymère.

3. Dérivé poly(4,5,9,10-tétrahydropyrèn-2,7-diyle) selon la revendication 1, caractérisé en ce qu'il est un copolymère.

4. Dérivés poly(4,5,9,10-tétrahydropyrèn-2,7-diyle) selon une ou plusieurs des revendications 1 à 3, caractérisés en ce que dans la formule (I) R² et R⁴ représentent un atome d'hydrogène.

5. Dérivés poly(4,5,9,10-tétrahydropyrèn-2,7-diyle) selon une ou plusieurs des revendications 1 à 4, caractérisés en ce que dans la formule (I)
R² et R⁴ représentent un atome d'hydrogène et
R¹ et R³ sont identiques et représentent un groupe alkyle linéaire ou ramifié ayant 1 à 22 atomes de carbone, dans lequel un ou plusieurs groupes CH₂ non voisins peuvent même être remplacés par -O-, -COO-, -OOC- et/ou un groupe phénylène, des groupes aryle ou aryloxy, le composé aromatique pouvant être substitué avec un groupe alkyle en C₁-C₂₂, alcoxy en C₁-C₂₂, Br, Cl, F, CN, et/ou NO₂, représentent un atome de brome, de chlore, de fluor, un groupe CN, NO₂, ou carboalcoxy avec 2 à 23 atomes de carbone;
n est 10 à 150.

6. Procédé pour la préparation de composés de formule (I) selon une ou plusieurs des revendications 1 à 5, caractérisé en ce qu'on met à réagir
un ou plusieurs composés de formule (II), 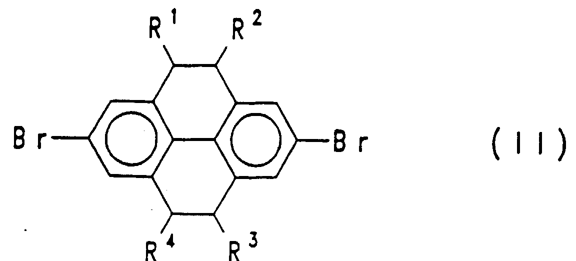 dans laquelle les symboles ont des significations identiques à celles de la formule (I) dans la revendication 1, dans un solvant organique ou un mélange de solvants organiques inerte avec le bis(1,5-cyclooctadiène)nickel(0) et une 2,2'-bipyridine.

7. Utilisation de dérivés poly(4,5,9,10-tétrahydropyrèn-2,7-diyle) selon une ou plusieurs des revendications 1 à 5 comme matériaux électroluminescents.

8. Matériau électroluminescent, contenant un ou plusieurs dérivés poly(4,5,9,10-tétrahydropyrèn-2,7-diyle) selon une ou plusieurs des revendications 1 à 5.

9. Dispositif électroluminescent avec une ou plusieurs couches actives, caractérisé en ce qu'au moins une de ces couches actives contient comme matériau électroluminescent un dérivé poly(4,5,9,10-tétrahydropyrèn-2,7-diyle) selon une ou plusieurs des revendications 1 à 5.

10. Procédé de préparation de dérivés 2,7-dibromo-4,5,9,10-tétrahydropyrène,caractérisé en ce qu'on met à réagir avec du brome un dérivé du 4,5,9,10-tétrahydropyrène dans un solvant organique inerte en présence d'un métal du sous groupe huit sur un support en charbon actif.
